# EUROPEAN PATENT SPECIFICATION

(11) **EP 3 855 448 B1**
(45) Date of publication and mention of the grant of the patent: **30.10.2024**
(21) Application number: 20163217.1
(22) Date of filing: 16.03.2020
(51) Int. Cl.: G16H 40/40, G16H 40/63

(54) **RECORDING A STATE OF A MEDICAL DEVICE**
AUFZEICHNUNG EINES ZUSTANDS EINER MEDIZINISCHEN VORRICHTUNG
ENREGISTREMENT D'UN ÉTAT D'UN DISPOSITIF MÉDICAL

(30) Priority: 21.01.2020 US 202016747773
(43) Date of publication of application: 28.07.2021
(73) Proprietor: Fenwal, Inc., Lake Zurich, IL 60047 (US)
(72) Inventor: ROXAS, James Darren, Lake Zurich, IL 60047 (US); CASE, Brian C., Lake Zurich, IL 60047 (US); KRAUSE, Kevin, Lake Zurich, IL 60047 (US); MIN, Kyungyoon, Lake Zurich, IL 60047 (US); MOSKAL, Witold, Lake Zurich, IL 60047 (US); CORK, William, Lake Zurich, IL 60047 (US)
(74) Representative: Maikowski & Ninnemann Patentanwälte Partnerschaft mbB

(56) References cited:
- US-A1- 2009 012 449
- US-A1- 2017 039 423

## Description

### BACKGROUND

The present application relates generally to recording a state of a medical device. The present application relates more specifically to recording an image of a disposable component used with a medical device for diagnostic purposes.

### BRIEF DESCRIPTION OF THE DRAWINGS

FIG. 1 is a flowchart of a method of recording a state of a medical device, according to an illustrative embodiment;
FIG. 2 is a flowchart of a method of recording a state of a medical device, according to an illustrative embodiment;
FIG. 3 is a medical device for recording an image in response to a detected error condition, according to an illustrative embodiment;
FIG. 4 is a medical device for recording an image in response to a detected error condition, according to a second illustrative embodiment; and
FIG. 5 is a medical device for recording an image in response to a detected error condition, according to a third illustrative embodiment.

### DETAILED DESCRIPTION OF ILLUSTRATIVE EMBODIMENTS

In one embodiment, a medical device has a housing having an external surface, a disposable component disposed on the external surface, and a camera coupled to the medical device. The camera is configured to acquire an image representing the disposable component and at least a portion of the external housing. A programmed processing circuit is configured to operate the medical device using the disposable component, to detect an error condition on the medical device, and in response to the detection of the error condition, acquire an image from the camera. The image is stored in a memory of the medical device, wherein the memory is accessible for later retrieval of the image.

In some embodiments, the medical device is an apheresis device and the camera is coupled to the medical device in a position to image a plurality of lines of the disposable component and at least one blood product bag of the disposable component.

In some embodiments, the medical device comprises a user interface device coupled to the programmed processing circuit and the programmed processing circuit is configured to transmit the stored images in response to a user input provided via the user interface device.

In another embodiment, a method of recording a state of a medical device comprises operating the medical device to use the disposable component, detecting an error condition on the medical device, acquiring an image from a camera coupled to the medical device and storing the image in a memory of the medical device. The image represents the disposable component and at least a portion of the external housing of the medical device. The memory is accessible for later retrieval of the image.

The error condition may be a type of error condition that results in the processing circuit stopping the medical device operation.

In some embodiments, the medical device acquires the image for some types of error conditions but not for other types of error conditions.

The image or images can be pushed to or pulled from a remote computer for diagnostic purposes.

In some embodiments, the camera can further be used to detect improper installation of the disposable component.

In some embodiments, the error condition may be a detection of red blood cells at a plasma line hemoglobin sensor. In some embodiments, the error condition may be fluid detected on a pressure transducer.

In some embodiments, the image may be acquired automatically, without requiring or prompting for any manual user input.

In some embodiments, medical information about the state of the device is stored along with the acquired image. In some embodiments, the medical information comprises a device identifier and an indication of the operating state of the device.

In some embodiments, the image is acquired in response to and near in time to detection of an error condition, for example within less than 10 seconds. In some embodiments, the image is acquired before an error condition is detected and in response to a threshold being surpassed for a sensed value.

In some embodiments, the camera sensor is disposed on an arm extending horizontally over the external housing portion to be imaged.

In some embodiments, the camera has an orientation that is adjustable by a human operator.

In some embodiments, the camera is configured to image a plasma collection container comprising a blood line, a plasma line and a plasma bag.

In some embodiments, an external surface of the machine to be imaged faces substantially horizontally relative to a base or floor beneath the base. In some embodiments, the camera is disposed in a portion of the housing extending outward over the external surface.

In some embodiments, the camera sensor is disposed on a retractable arm coupled to the medical device housing.

In some embodiments, the medical device may be configured to video record the disposable component, portion of the external surface of the medical device, and/or other portions of the medical device and its surroundings.

A processing circuit may comprise analog and/or digital electrical components configured or programmed to perform any of the functions described herein, including drivers, buffers, amplifiers, etc. The processing circuit may comprise one or more microprocessors, microcontrollers, application-specific integrated circuits, programmable logic devices, etc., which may further be programmed by way of an operating system, applications, and/or other computer programs stored on a tangible memory device. A memory may comprise RAM, Flash, volatile and/or non-volatile memory of a variety of types used to support processing circuit in executing its functionalities.

A camera is configured to acquire light in the form of images and/or video and to provide the acquired image data to the processing circuit. Acquired images, video, and/or sounds may be stored in the memory and/or transmitted via a transceiver to a remote device, such as a desktop computer, laptop computer, or smartphone.

A transceiver coupled to the processing circuit may comprise one or more wired or wireless transceiver circuits configured to transmit and receive data between the medical device and other computing devices. The transceiver may comprise technology for wide area networks, local area networks, personal area networks, or other networking, such as communications according to a Bluetooth specification, an IEEE 802.11 specification, a Wi-Fi or Wi-Max specification, a cellular specification, a Universal Serial Bus specification, a near-field communication specification, etc.

An input device may comprise user input devices such as a push-button input, a touch pad input, a swipe input, hard or soft keys, etc.

Any of the teachings herein can be applied to a variety of medical devices and procedures. In some cases, these medical procedures may be procedures, such as non-invasive procedures, performed by a medical device suitably configured. One procedure is an apheresis procedure performed by an apheresis machine. Another procedure is an enteral feeding procedure performed by an enteral feeding pump. An enteral feeding pump is configured to pump nutrients at a controlled rate and amount. A non-invasive procedure may be an apheresis procedure performed by an apheresis machine using as blood source such as a container containing whole blood previously collected from a patient. The treated blood or blood component may subsequently be collected in a container for further use.

Certain examples provide mobile applications for medical devices including blood collection or apheresis devices, infusion pumps, drug delivery pumps, and/or other medical devices. For example, an infusion pump infuses fluids, medication, or nutrients.

A medical device may be a device that provides a medicament, extracts fluid or tissue, implants an object, or captures a medical image For example, the medical device may be a dialysis machine (e.g., a hemodialysis machine, a hemofiltration machine, etc.), an infusion pump, a drug delivery system, etc. The medical device may be an apheresis machine configured to draw blood from a whole blood source (e.g., a container with previously collected blood) and/or otherwise process blood components. In some implementations, the medical device may use measurements to control the medical procedure. The measurements may be taken directly by the medical device or may be received by the medical device via a data link or communication link from a measurement device.

A remote computer may comprise a server computer which may be any form of computing device or set of computing devices configured to store and communicate electronic data. For example, the server may be a personal computer, a mainframe, a cloud-computing environment, or a data center. The server may include a processing circuit that includes a processor and a memory that stores instructions for processor. The server may also include an interface circuit configured to communicate with a network via a wireless or hardwired connection, according to various implementations.

A network may be any form of computer network that relays information between a medical device, remote computer or server, etc. For example, a network may include the Internet and/or other types of data networks, such as a local area network (LAN), a wide area network (WAN), a cellular network, satellite network, or other types of data networks. The network may also include any number of intermediary computing devices (e.g., computer, servers, routers, network switches, etc.) that are configured to receive and/or transmit data within the network.

The server may receive and store data generated by the medical device and/or operational data generated by the medical device and stored in its memory. The server may be configured to communicate with the medical device via any number of different networking protocols. For example, the server may communicate with the medical device via an HTTP connection, FTP connection, SSH connection, a telnet connection, combinations thereof, or other similar networking protocols. In some implementations, the server may relay data between the medical device and another electronic device. For example, the server may be a device that communicates with the medical device within the same medical facility and relays information between the medical device and a server of the manufacturer of the medical device via the Internet.

In some embodiments, a camera may be disposed to acquire an image of at least a portion of the medical device. The camera may be configured to acquire an image of the medical device in response to user input at a user input device (e.g., a touch pad, a hard key, a soft key, etc.). The user may choose to acquire an image after seeing an error message or other notification on a screen of the medical device, after hearing an audible alarm, etc. Alternatively, the camera may automatically, without requiring user input, acquire one or more images or video of the medical device at periodic or intermittent times, continuously, or when an alert, alarm or other condition is detected by the medical device. A processing circuit of the medical device may be configured to detect a condition of the medical device from an acquired image and to generate input data used to retrieve an instruction relating to the medical device from a remote computer. The processing circuit may then generate a request message comprising one or more of these determined data and transmit the request message to a remote computer using its wireless transceiver to request information.

Upon identification by the medical device of an error, alarm, or other issue, or upon request of a user or receipt of other input data (e.g., from another computer such as the remote computer), the processing circuit of the medical device may be configured to retrieve from a memory an instruction relating to the medical device based on the input data, and to display the instruction relating to the medical device on the display of the medical device. The memory may be local to the medical device or remote (e.g., a server-based memory, cloudbased memory, etc.). The instruction may comprise a plurality of instructions, e.g., step-by-step instructions, displayed and/or heard by a user for procedures, service of the instrument, programming the instrument, entering a test mode of the instrument, responding to alarms, how to program the device, etc.

The instruction may relate to troubleshooting a problem encountered with the medical device, which may comprise one or more instructions to the operator of the device to try different actions with the device to discover and/or solve or address the problem. The instruction may instruct a user how to respond to an alarm or alert generated by the medical device.

In one embodiment, the medical device is disposed in a medical facility, which may be a blood donation facility, wherein a medical product comprises a blood donation kit. A blood donation kit may comprise a blood bag, tubing and/or other components that are configured for use with a whole blood source ) for a single blood donation and may be considered to be disposable.

In some embodiments, a medical device may be configured to determine a characteristic of the medical device using the camera and to compare the characteristic with predetermined data for a component of the medical device. The camera may be configured to acquire an image of a component of the medical device with a camera, to process the image to identify a characteristic of the component, to compare the characteristic to predetermined data for the component, and to generate output data based on the comparison. The output data may be an instruction, alert, warning, verification, screen alert, audible alert, haptic feedback, vibration of device, etc. The output data may further trigger or comprise the display of directions on the medical device for correcting information (e.g., programming an apheresis or infusion, etc.).

In some embodiments, the component imaged is a disposable component configured to be loaded into the medical device, such as a blood donation kit having tubing and a blood bag and optionally a cartridge. In this embodiment, the characteristic of the disposable component is an indication of how the component is loaded into the medical device and wherein the predetermined data is data representing a proper loading of the component. For example, the medical device may provide kit loading verification (e.g., visual identification to ensure proper loading of kits before a procedure is commenced). For example, one or more codes, marks, or indicators may be printed on the medical device that would be covered by portions of the disposable component (or on the disposable component that would be covered by the medical device) when the component is properly inserted. The medical device may use its camera to look for those marks and, finding none, conclude that the component is properly installed. Other image processing techniques may be used to identify proper installation of a disposable kit.

In various embodiments, the component of the medical device being scanned, imaged, or viewed may be a disposable component used in a medical procedure, a replaceable component, a tubing, a source of a drug, a blood component receptacle or bag, a cartridge, or other components of various medical devices.

The medical device information acquired by the camera may be a video of the medical device acquired for a predetermined period of time (e.g., less than 5 seconds, less than 30 seconds, etc.) as seen by the camera.

The component may be a disposable or non-disposable component of the medical device, such as, a cartridge, tubing and one or more blood bags. A disposable component may be a single-use component intended to be used on a single whole blood source and/or for a single procedure and thereafter disposed of.

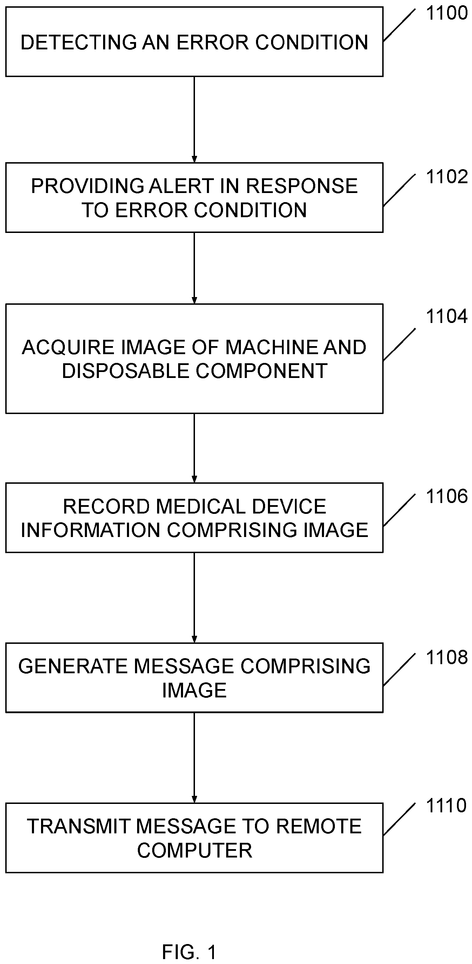

Referring now to FIG. 1, a method of recording a state of an apheresis machine having a disposable component disposed thereon will be described. As described herein, some medical devices, such as apheresis machines, may use a disposable component which is installed on the apheresis device by a human operator and is intended to be disposed of after use for a single procedure or multiple procedures. In some embodiments, the state of the apheresis machine can be recorded by visual image to show whether the machine is in a state having a properly installed disposable component or is in a state of having an improperly installed disposable component. Other states of the machine or aspects of the machine's state can be recorded, such as alarms or alerts displayed on the display, the display screen being shown at the time, the presence or absence of one or more fluids in the lines and/or bags of the disposable component, etc.

At block 1100, the medical device is configured to detect an error condition on the apheresis device. The error condition can be detected by a programmed processing circuit (e.g., microprocessor, microcontroller, control circuit, application-specific integrated circuit, or other compilation of analog and/or digital circuit components, etc.) running an operating algorithm or program which continuously, periodically, or intermittently monitors certain inputs from the machine, such as sensors, detectors, motor currents, user inputs, etc. The error condition may be a significant alarm issue that the instrument detected, particularly in a situation of an adverse event. In one example, the significant alarm issue may be the detection of red blood cells at a plasma line hemoglobin sensor leading to a plasma collection. In another example, the significant alarm issue may be fluid detection on a pressure transducer. In another example, the significant alarm issue may be blood leak detection on the disposable component. In response to the detection of the error condition, the processing circuit of the medical device may be configured (at block 1102) to provide at least one of an audible alert and a visual alert to the human operator. The processing circuit may also be configured (at block 1104) to acquire with a camera an image of a portion of the apheresis machine having the disposable component. The image may be acquired automatically without requiring user input. In alternative embodiments, the user may be prompted to request the device acquire an image by way of pressing a user input device (e.g., a button on a touch screen, a hard key, a softkey, a voice command, etc.). In some embodiments, the detection of the error condition sets into motion the acquisition and recording of the image without requiring or prompting for any manual user input.

At block 1106, certain medical device information may be stored, such as the image, a device identifier, status information about the device (e.g., whether it was running or idle, the particulars of user inputs, the step or stage in a collection process, etc.), make, model or type of medical device, etc. At a block 1108, the processing circuit may be configured to generate a message in a communication format and (at block 1110), the message may be transmitted over a network to a computer remote from the medical device (e.g., in another room of a facility, in another building of a campus, or in another city, state, country, etc.). The storage, generating and transmission may occur automatically, in response to the triggering event of the error condition being detected. Alternatively, one or more of the steps may require user input or confirmation in alternate embodiments before the step is executed.

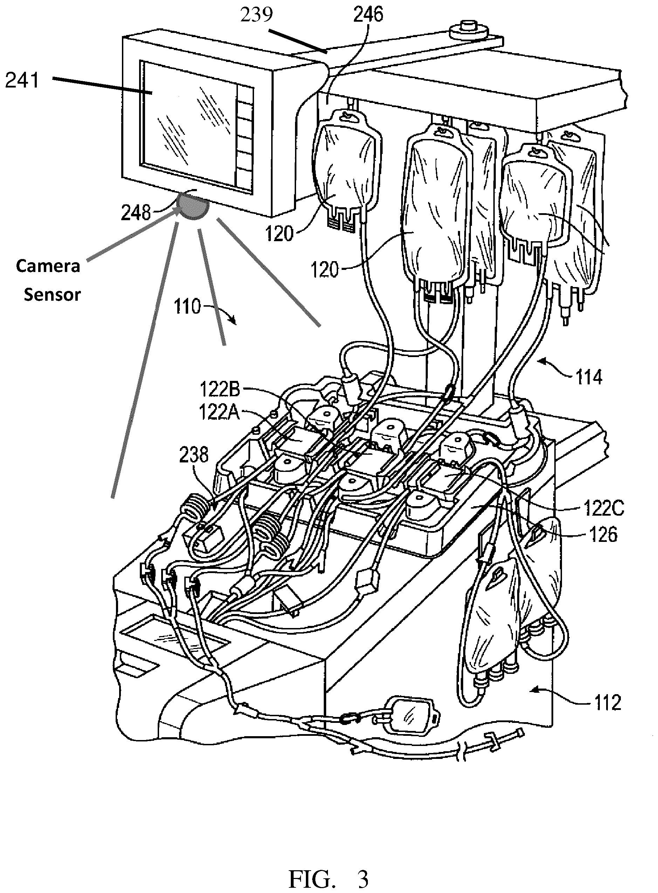

FIG. 3 provides an illustration of an apheresis machine 110 having a disposable component 114, according to an exemplary embodiment. As can be seen, disposable component 114 comprises a plurality of lines or tubes interconnecting several bags 120. Bags may be provided as a source of anticoagulant, saline or other additives. Bags may be alternatively be provided to collect blood components, such as plasma, red blood cells, white blood cells, etc.

A human operator is tasked with loading the apheresis machine 110 with the disposable component 114 and aligning the tubes or lines with pumps, sensors, channels, and other components of the apheresis machine. It is possible that the human operator improperly installs the disposable component, which can lead to error conditions during operation of the machine. When diagnosing the cause of an error condition (e.g., red plasma, noise, brown urine, etc.), it is difficult to investigate the root cause without information about the state of the machine and its disposable component. Further, if the disposable component is removed, the arrangement has been disturbed which also may make it difficult to identify the cause of the error.

As shown, a camera or camera sensor is coupled to a portion of a housing 248 of the medical device 114. The camera may be disposed in a predetermined orientation such that an image or images of the disposable component and an external surface of the apheresis machine housing can be acquired. In some embodiments, by acquiring the images in response to, near in time to, or immediately after the error condition has been detected, useful information about the state of the machine and potential cause of the error condition can be recorded or retained for future analysis. The camera sensor may be configured to log images for every significant alarm detected by the instrument and stored for later retrieval either in batch form or individually. In some embodiments, images and/or video may be acquired even before an error condition is detected, for example in response to a threshold being surpassed for a sensed value that is not yet at an alarm state but indicative of a potential future alarm.

In this case, an external housing portion 238 faces substantially upward toward the camera sensor, making it easier to acquire a useful image of the state of the machine. The camera sensor may be disposed on an arm 239 extending horizontally over the external housing portion 238. The camera sensor may be coupled to a user interface housing portion 248 of the machine. Other configurations are contemplated. The camera sensor may have imaging hardware installed within the camera, such as a charge-coupled device, a complementary metal-oxide-semiconductor device, or other imaging technology. The camera sensor may be configured to sense light waves of different ranges along the electromagnetic spectrum, such as visible light, invisible light, infrared light, ultraviolet light, or radiation of other wavelength ranges. For example, sensing infrared light may provide information relating to the temperature of fluids within the tubing of the disposable component. Acquiring an image of visible light and an image of infrared light may be provide more information that is useful in diagnosing the cause of an error condition.

The error condition may be any of a number of conditions. For example, the error condition may be an indication of a failure of a component or a processing step. In response to an error condition, the machine may be configured to display on user interface 241 contact information for a manufacturer of the medical machine to a human operator. The user interface 241 may also prompt a user to instruct the device to send diagnostic information, including any images acquired, to a service technician's computer via a network.

In some embodiments, an image or images (or video) may be acquired and stored or logged for later retrieval. In one embodiment, the camera may have a position and/or orientation that is adjustable by a human operator. The camera may have a controllable zoom, focus, tint, brightness, and/or other digital image editing features controllable from a user interface of the medical machine. Images may be transmitted manually or automatically over a network to a predetermined destination (e.g., an IP address, an email address, or other network location), or the user interface may be used for the human operator to enter a location address for the remote destination computer. The user interface may allow the user to select from among a plurality of images and/or other medical device information to send. Alternatively, the medical device may be programmed to send all images or predetermined medical device information without regard to user input.

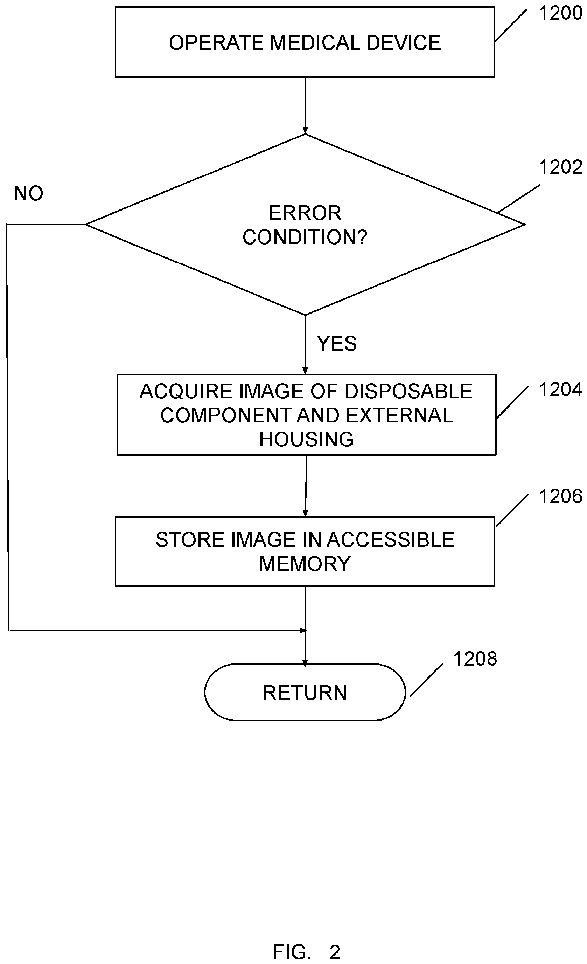

Referring now to FIG. 2, a method of recording a state of a medical device will be described according to another embodiment. The medical device may comprise a programmed processing circuit and a disposable component disposed on or installed on an external surface of a medical device housing. At a block 1200, the medical device is operated, for example in a preparation step, a priming step, a blood processing step, a blood return step, a procedure finalization step, or other steps, such as providing a software update to the machine, running machine diagnostics, etc. In some embodiments, the device operation of block 1200 involves use of the disposable component.

At a block 1202, the medical device is configured to detect an error condition on the medical device using the processing circuit. The processing circuit, in response to the detection of the error condition, may be configured to acquire an image (block 1204) from a camera coupled to or built-in to the medical device, the image representing the disposable component and at least a portion of the external housing of the medical device. The image may be acquired directly in response to detection of the error, or the acquisition may be done based at least in part on the detection of the error and based on other inputs (e.g., such as a user confirmation to acquire the image). The image may be acquired immediately after the detection of the error or shortly after detection of the error (e.g., within less than 3 seconds of the error, within less than 10 seconds of the error, within less than 30 seconds of the error, etc.). The image or video may be acquired even before detection of the error. The processing circuit may be programmed with threshold values for each sensor on the instrument to trigger an alarm. The processing circuit may further be programmed to start acquiring images and/or video when monitored sensor data reach certain values or thresholds before reaching the threshold value to trigger an alarm.

At a block 1206, the processing circuit is configured to store the image in a memory of the medical device, wherein the memory is accessible for later retrieval of the image. The memory may be a flash drive, a USB memory stick, random access memory, flash EPROM, EEPROM, hard disk storage, solid state memory, or other memory types. The memory may have an interface for retrieval of the image, images or other medical device information, such as an interface bus to a communications network, an interface circuit to a USB memory card slot, an interface to an SD card slot, or other interfaces.

The medical device may be configured to only acquire an image (or only acquire an image automatically) when an error condition of a certain significance or severity is detected. For example, a processing circuit may detect error conditions of a first type (e.g., more significant) and error conditions of a second type (less significant). The processing circuit may acquire the image for errors of the first type and may be configured to not acquire the image for error conditions of the second type. In some examples, the error conditions of the first type may also result in the processing circuit stopping the medical procedure.

The image may be transmitted to a remote computing device without requiring input from a human operator, e.g., automatically. In alternative embodiments, the image may be transmitted only after user input to confirm the transmission. In some embodiments, the image may be transmitted over the network in response to a request message received from the remote computer over the computer network. For example, the message may be formatted as an HTTP response message and the remote computer may act as a TCP/IP client requesting the message from the medical machine operating as a TCP/IP server computer.

In some embodiments, the camera and processing circuit may be configured to acquire images for transmission as described herein and also may be configured in another mode or use to detect improper installation of the disposable component. If the camera and processing circuit detect improper installation (e.g., using image processing techniques), the processing circuit may be configured to provide an audible and/or visual alert to a user interface of the medical device in response to detection of improper installation of the disposable component.

One or more messages described herein may be transmitted to a manufacturer of the medical device and/or the disposable component. The manufacturer may operate a computer configured to aggregate data from multiple machines at different facilities in different locations operated by different companies. The aggregated data can be processed and analyzed to determine common error conditions, frequent errors in installing disposable components, etc., in order to guide future training or troubleshooting processes.

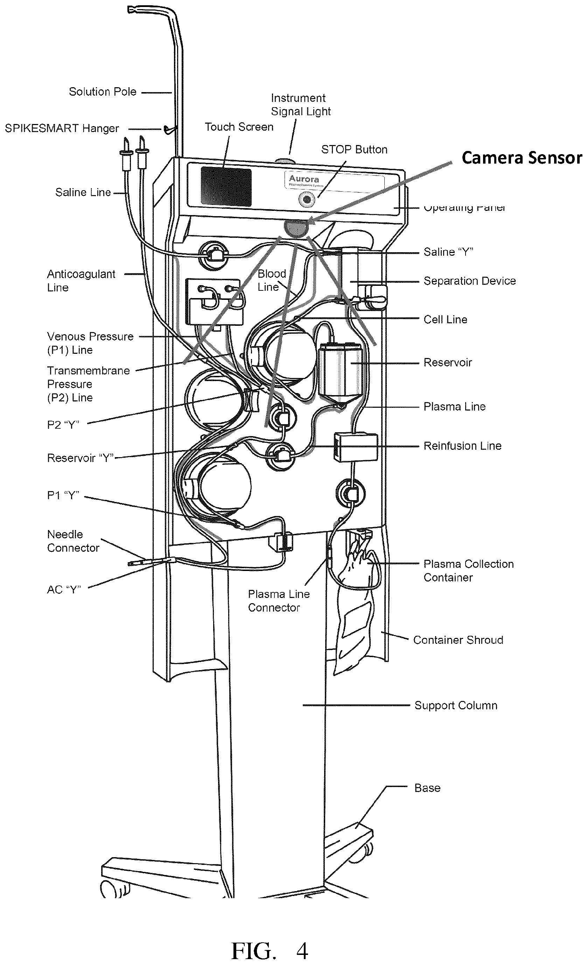

FIG. 4 illustrates another medical instrument, a plasmapheresis machine. This plasmapheresis machine uses a disposable component comprising a plasma collection container, a plasma line connector, a plasma line, a cell line, a blood line, an anticoagulant line, a needle connector, and other components. The plasmapheresis machine comprises a touch screen for user interface functions, a signal light for outputting visual indications, a stop button hard key for stopping the machine, and other components. In this embodiment, an external surface of the machine that interfaces with the distributed component faces substantially horizontally relative to a base or floor beneath the base. A camera sensor is disposed in a portion of the housing extending outward over the external surface. The portion of the housing comprises the user interface, stop button and signal light, and a bottom surface thereof may be coupled to the camera sensor. The camera sensor has a field of view directed downward in a configuration to capture an image or images of portions of the disposable component as well as pumps and other components of the plasmapheresis machine. In one embodiment, the medical instrument may have a retractable arm dedicated to the camera sensor, the retractable arm configured to manually or automatically extend a predetermined distance away from the housing and outward at an angle having a better view coverage of the disposable installed on the instrument. In one embodiment, the arm automatically extends in response to an error condition to acquire images and/or video of the disposable arrangement.

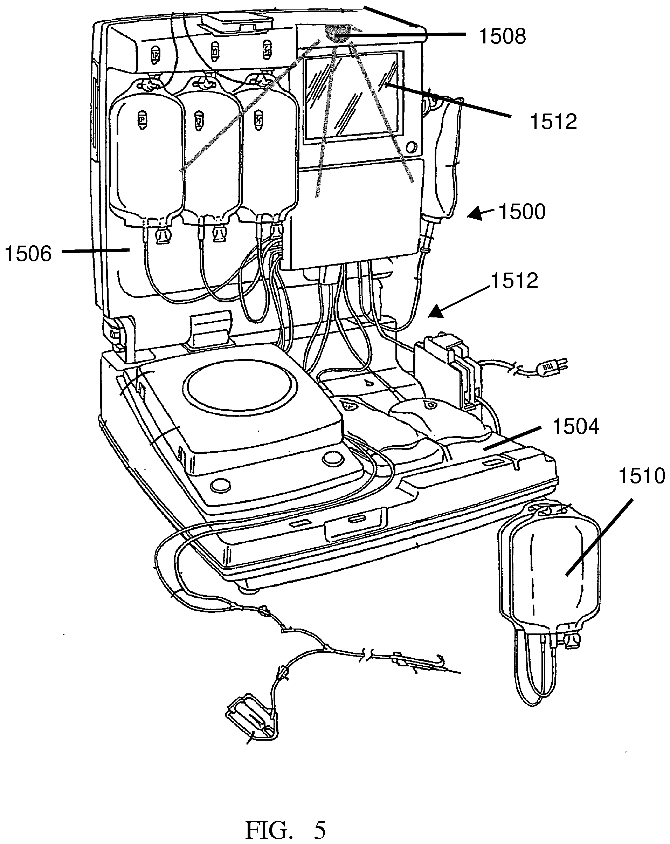

Referring now to FIG. 5, a medical device 1500 comprises a medical device housing having a first housing portion 1506 facing substantially horizontally and hinged to a second housing portion 1504 facing substantially vertically, when in a user or operating configuration. In this embodiment, a camera 1508 may be configured to image one or both of housing portion external surfaces 1506, 1504. Camera 1508 may also be configured to image a disposable component 1512 disposed on one or both external surfaces of the medical device housing.

Medical device 1500 may further comprise a programmed processing circuit (internal to the housing) configured to operate the medical device to provide a medical procedure (such as plasmapheresis, red blood cell collection, both procedures, or other procedures) using the disposable component. The processing circuit may be configured to detect an error condition on the medical device 1500 and, in response to the detection of the error condition, acquire an image from camera 1508. The processing circuit may further be configured to store the image in a memory of the medical device (internal to the housing), wherein the memory is accessible for later retrieval of the image.

As shown, camera 1508 may be disposed in a position on the housing to image a plurality of lines of the disposable component and at least one blood product bag 1510 of the disposable component.

In some embodiments, the programmed processing circuit may be configured to store a plurality of images, each image acquired in response to a detection of an error condition.

In some embodiments, the medical device may further comprise a network interface circuit configured to transmit the stored images over a network to a remote computer. A network interface circuit may comprise hardware, software, firmware components, etc., configured to format messages for bidirectional communication over a network, such as an Ethernet or other network, local area, wide area, Bluetooth network, Wi-Fi network, etc.

In some embodiments, a user interface device 1513 may be coupled to the programmed processing circuit. The programmed processing circuit may be configured to transmit stored images in response to a user input provided via the user interface device. For example, user interface device 1513 may be a touch screen. The processing circuit may be configured to prompt a user in the event of an error condition with a message such as "ACQUIRE DIAGNOSTIC INFORMATION?" or "READY TO ACQUIRE IMAGE?" or another prompt. A "CONFIRM" or "OK" input key may be provided by the touch screen for user input to instruct the processing circuit to acquire the image. Similar configurations can be made to solicit user instruction to transmit the image or other medical device information, select a destination for the transmissions, etc.

In another embodiment, the medical device may be configured to video record the disposable component, portion of the external surface of the medical device, and/or other portions of the medical device and its surroundings. The video may be recorded of an entire procedure, or of portions of the procedure beyond those relating to an error condition. For example, upon beginning a new procedure, the medical device may be configured to begin recording the operator's installation of the disposable component, the use of the disposable component during the procedure, and the removal of the disposable component. This video may be stored in memory for later retrieval if needed. The procedure may be automatically erased after a period of time (e.g., 1 day, 5 days, 30 days, 90 days, etc.). Advantageously, in the case of an adverse event, or for other purposes, the entire procedure from beginning to end may be viewed for investigation.

While illustrative embodiments have been described herein, it will be apparent from the disclosure of varied embodiments that modifications and alternatives are contemplated in various different embodiments. For example, the teachings herein may be applied to other medical devices using disposable components, such as infusion pumps using disposable syringes or medicament bags, feeding pumps using disposable nutrient bags, etc. Also, while detected error conditions are described as triggering image acquisition, other process steps of medical machine may trigger automatic acquisition of an image, such as the completion of a step of a medical procedure (e.g., a final step, an intermediate step, etc.), or other triggering events.

## Claims

1. A medical device (110, 1500), comprising:
a medical device housing (248) having an external surface (238, 1504, 1506);
a disposable component (114, 1512) disposed on the external surface (238, 1504, 1506) of the medical device housing (248), wherein the disposable component (114, 1512) is configured to be installed on the external surface (238, 1504, 1506) by a human operator and removable therefrom for disposal;
a camera (1508) coupled to the medical device housing and configured to acquire an image representing the disposable component (114, 1512) and at least a portion of the external surface (238); and
a programmed processing circuit configured to:
operate the medical device using the disposable component (114, 1512),
detect an error condition on the medical device (110, 1500),
**characterized in that** the programmed processing circuit is further configured to:
in response to the detection of the error condition, acquire an image from the camera (1508); and
store the image in a memory of the medical device (110, 1500), wherein the memory is accessible for later retrieval of the image.

2. The medical device of Claim 1, wherein the medical device is an apheresis device, the camera coupled to the medical device housing in a position to image a plurality of lines of the disposable component and at least one blood product bag (120, 1510) of the disposable component (114, 1512).

3. The medical device of Claim 2, wherein the programmed processing circuit is configured to store a plurality of images, each image acquired in response to a detection of an error condition.

4. The medical device of Claim 3, further comprising a network interface circuit configured to transmit the stored images over a network to a remote computer.

5. The medical device of Claim 4, further comprising a user interface device (241, 1513) coupled to the programmed processing circuit, wherein the programmed processing circuit is configured to transmit the stored images in response to a user input provided via the user interface device (241, 1513).

6. A method of recording a state of a medical device (110, 1500) having a programmed processing circuit and a disposable component (114, 1512) disposed on an external surface (238, 1504, 1506) of a medical device housing (248), wherein the disposable component (114, 1512) is configured to be installed on the external surface (238, 1504, 1506) by a human operator and removable therefrom for disposal, comprising:
operating (1200) the medical device to provide a medical procedure using the disposable component;
detecting an error condition (1100, 1202) on the medical device using the processing circuit **characterized in that** the processing circuit, in response to the detection of the error condition, acquires an image (1104, 1204) from a camera coupled to the medical device housing, the image representing the disposable component and at least a portion of the external housing of the medical device; and
storing the image (1106, 1206) in a memory of the medical device, wherein the memory is accessible for later retrieval of the image.

7. The method of Claim 6, wherein the error condition results in the processing circuit stopping the medical procedure.

8. The method of Claim 6, the processing circuit detecting error conditions of a first type and error conditions of a second type, wherein the processing circuit acquires the image for errors of the first type and does not acquire the image for error conditions of the second type.

9. The method of Claim 8, further comprising providing (1102) at least one of a visual and audible alarm on a user interface of the medical device in response to the error conditions of the first type and the error conditions of the second type.

10. The method of Claim 6, further comprising transmitting the image (1110) stored in the memory over a network to a remote computing device without requiring input from a human operator.

11. The method of Claim 6, further comprising transmitting the image (1110) stored in the memory over a network to a remote computing device in response to input from a human operator.

12. The method of Claim 6, further comprising receiving a request for the image over a network at the processing circuit from a remote computer and, in response, transmitting the image over the network to the remote computer.

13. The method of Claim 6, further comprising using the camera and processing circuit to detect improper installation of the disposable component and providing an audible and/or visual alert to a user interface of the medical device in response to detection of improper installation of the disposable component.

## Patentansprüche

1. Medizinische Vorrichtung (110, 1500), umfassend:
ein medizinisches Vorrichtungsgehäuse (248) mit einer Außenoberfläche (238, 1504, 1506);
eine Einwegkomponente (114, 1512), die auf der Außenoberfläche (238, 1504, 1506) des medizinischen Vorrichtungsgehäuses (248) angeordnet ist, wobei die Einwegkomponente (114, 1512) ausgestaltet ist, um durch eine menschliche Bedienungsperson auf der Außenoberfläche (238, 1504, 1506) installiert zu werden und von dieser zur Entsorgung entfernbar zu sein;
eine Kamera (1508), die an das medizinische Vorrichtungsgehäuse gekoppelt ist und ausgestaltet ist, um ein Bild zu erfassen, das die Einwegkomponente (114, 1512) und mindestens einen Anteil der Außenoberfläche (238) repräsentiert; und
eine programmierte Verarbeitungsschaltung, die ausgestaltet ist zum:
Bedienen der medizinischen Vorrichtung unter Verwendung der Einwegkomponente (114, 1512),
Detektieren eines Fehlerzustands an der medizinischen Vorrichtung (110, 1500), **dadurch gekennzeichnet, dass** die programmierte Verarbeitungsschaltung des Weiteren ausgestaltet ist zum:
Erfassen eines Bildes durch die Kamera (1508) in Reaktion auf die Detektierung des Fehlerzustands; und
Speichern des Bildes in einem Speicher der medizinischen Vorrichtung (110, 1500), wobei der Speicher zum späteren Abrufen des Bildes zugänglich ist.

2. Medizinische Vorrichtung nach Anspruch 1, wobei die medizinische Vorrichtung eine Apheresevorrichtung ist, und die Kamera in einer Position, um eine Vielzahl von Linien der Einwegkomponente und mindestens einen Blutproduktbeutel (120, 1510) der Einwegkomponente (114, 1512) abzubilden, an das medizinische Vorrichtungsgehäuse gekoppelt ist.

3. Medizinische Vorrichtung nach Anspruch 2, wobei die programmierte Verarbeitungsschaltung ausgestaltet ist, um eine Vielzahl von Bildern zu speichern, wobei jedes Bild in Reaktion auf eine Detektierung eines Fehlerzustands erfasst wurde.

4. Medizinische Vorrichtung nach Anspruch 3, des Weiteren umfassend eine Netzwerkschnittstellenschaltung, die ausgestaltet ist, um die gespeicherten Bilder über ein Netzwerk an einen Remote-Computer zu übermitteln.

5. Medizinische Vorrichtung nach Anspruch 4, des Weiteren umfassend eine Benutzerschnittstellenvorrichtung (241, 1513), die an die programmierte Verarbeitungsschaltung gekoppelt ist, wobei die programmierte Verarbeitungsschaltung ausgestaltet ist, um die gespeicherten Bilder in Reaktion auf eine Benutzereingabe zu übermitteln, die mittels der Benutzerschnittstellenvorrichtung (241, 1513) bereitgestellt wurde.

6. Verfahren zum Aufzeichnen eines Zustands einer medizinischen Vorrichtung (110, 1500), die eine programmierte Verarbeitungsschaltung und eine Einwegkomponente (114, 1512), die auf einer Außenoberfläche (238, 1504, 1506) des medizinischen Vorrichtungsgehäuses (248) angeordnet ist, aufweist, wobei die Einwegkomponente (114, 1512) ausgestaltet ist, um durch eine menschliche Bedienungsperson auf der Außenoberfläche (238, 1504, 1506) installiert zu werden und von dieser zur Entsorgung entfernbar zu sein, umfassend:
Bedienen (1200) der medizinischen Vorrichtung zur Bereitstellung eines medizinischen Eingriffs unter Verwendung der Einwegkomponente;
Detektieren eines Fehlerzustands (1100, 1202) an der medizinischen Vorrichtung unter Verwendung der Verarbeitungsschaltung, **dadurch gekennzeichnet, dass** die Verarbeitungsschaltung in Reaktion auf die Detektierung des Fehlerzustands ein Bild (1104, 1204) durch eine Kamera erfasst, die an das medizinische Vorrichtungsgehäuse gekoppelt ist, wobei das Bild die Einwegvorrichtung und mindestens einen Anteil des Außengehäuses der medizinischen Vorrichtung repräsentiert; und
Speichern des Bildes (1106, 1206) in einem Speicher der medizinischen Vorrichtung, wobei der Speicher zum späteren Abrufen des Bildes zugänglich ist.

7. Verfahren nach Anspruch 6, wobei der Fehlerzustand dazu führt, dass die Verarbeitungsschaltung den medizinischen Eingriff stoppt.

8. Verfahren nach Anspruch 6, wobei die Verarbeitungsschaltung Fehlerzustände eines ersten Typs und Fehlerzustände eines zweiten Typs detektiert, wobei die Verarbeitungsschaltung das Bild für Fehler des ersten Typs erfasst und das Bild für Fehlerzustände des zweiten Typs nicht erfasst.

9. Verfahren nach Anspruch 8, des Weiteren umfassend Bereitstellen (1102) von mindestens einem von einem visuellen und hörbaren Alarm an einer Benutzerschnittstelle der medizinischen Vorrichtung in Reaktion auf die Fehlerzustände des ersten Typs und die Fehlerzustände des zweiten Typs.

10. Verfahren nach Anspruch 6, des Weiteren umfassend Übermitteln des im Speicher gespeicherten Bildes (1110) über ein Netzwerk an eine Remote-Rechenvorrichtung, ohne dass eine Eingabe von einer menschlichen Bedienungsperson erforderlich ist.

11. Verfahren nach Anspruch 6, des Weiteren umfassend Übermitteln des im Speicher gespeicherten Bildes (1110) über ein Netzwerk an eine Remote-Rechenvorrichtung in Reaktion auf eine Eingabe von einer menschlichen Bedienungsperson.

12. Verfahren nach Anspruch 6, des Weiteren umfassend Empfangen einer Anforderung des Bildes über ein Netzwerk an der Verarbeitungsschaltung von einem Remote-Computer und in Reaktion darauf Übermitteln des Bildes über das Netzwerk an den Remote-Computer.

13. Verfahren nach Anspruch 6, des Weiteren umfassend Verwenden der Kamera und der Verarbeitungsschaltung zum Detektieren von fehlerhafter Installation der Einwegkomponente und Bereitstellen eines hörbaren und/oder visuellen Alarms an einer Benutzerschnittstelle der medizinischen Vorrichtung in Reaktion auf die Detektierung der fehlerhaften Installation der Einwegkomponente.

## Revendications

1. Dispositif médical (110, 1500), comprenant :
un boîtier de dispositif médical (248) présentant une surface externe (238, 1504, 1506) ;
un composant jetable (114, 1512) disposé sur la surface externe (238, 1504, 1506) du boîtier de dispositif médical (248), le composant jetable (114, 1512) étant configuré pour être installé sur la surface externe (238, 1504, 1506) par un opérateur humain et retiré de celle-ci pour être éliminé ;
une caméra (1508) couplée au boîtier de dispositif médical et configurée pour acquérir une image représentant le composant jetable (114, 1512) et au moins une partie de la surface externe (238) ; et
un circuit de traitement programmé configuré pour :
actionner le dispositif médical à l'aide du composant jetable (114, 1512),
détecter une condition d'erreur sur le dispositif médical (110, 1500), **caractérisé en ce que** le circuit de traitement programmé est en outre configuré pour :
en réponse à la détection de la condition d'erreur, acquérir une image à partir de la caméra (1508) ; et
stocker l'image dans une mémoire du dispositif médical (110, 1500), la mémoire étant accessible pour une récupération ultérieure de l'image.

2. Dispositif médical selon la revendication 1, le dispositif médical étant un dispositif d'aphérèse, la caméra étant couplée au boîtier de dispositif médical dans une position pour imager une pluralité de lignes du composant jetable et au moins une poche de produit sanguin (120, 1510) du composant jetable (114, 1512).

3. Dispositif médical selon la revendication 2, le circuit de traitement programmé étant configuré pour stocker une pluralité d'images, chaque image étant acquise en réponse à une détection d'une condition d'erreur.

4. Dispositif médical selon la revendication 3, comprenant en outre un circuit d'interface réseau configuré pour transmettre les images stockées sur un réseau à un ordinateur distant.

5. Dispositif médical selon la revendication 4, comprenant en outre un dispositif d'interface utilisateur (241, 1513) couplé au circuit de traitement programmé, le circuit de traitement programmé étant configuré pour transmettre les images stockées en réponse à une entrée utilisateur fournie par l'intermédiaire du dispositif d'interface utilisateur (241, 1513).

6. Procédé d'enregistrement d'un état d'un dispositif médical (110, 1500) comportant un circuit de traitement programmé et un composant jetable (114, 1512) disposé sur une surface externe (238, 1504, 1506) d'un boîtier de dispositif médical (248), le composant jetable (114, 1512) étant configuré pour être installé sur la surface externe (238, 1504, 1506) par un opérateur humain et en être retiré pour être éliminé, comprenant :
l'actionnement (1200) du dispositif médical pour fournir une procédure médicale à l'aide du composant jetable ;
la détection d'une condition d'erreur (1100, 1202) sur le dispositif médical à l'aide du circuit de traitement, **caractérisé en ce que** le circuit de traitement, en réponse à la détection de la condition d'erreur, acquiert une image (1104, 1204) à partir d'une caméra couplée au boîtier de dispositif médical, l'image représentant le composant jetable et au moins une partie du boîtier externe du dispositif médical ; et
le stockage de l'image (1106, 1206) dans une mémoire du dispositif médical, la mémoire étant accessible pour une récupération ultérieure de l'image.

7. Procédé selon la revendication 6, la condition d'erreur entraînant l'arrêt de la procédure médicale par le circuit de traitement.

8. Procédé selon la revendication 6, le circuit de traitement détectant des conditions d'erreur d'un premier type et des conditions d'erreur d'un second type, le circuit de traitement faisant l'acquisition de l'image pour des erreurs du premier type et ne faisant pas l'acquisition de l'image pour des conditions d'erreur du second type.

9. Procédé selon la revendication 8, comprenant en outre la fourniture (1102) d'au moins une parmi des alarmes visuelle et sonore sur une interface utilisateur du dispositif médical en réponse aux conditions d'erreur du premier type et aux conditions d'erreur du second type.

10. Procédé selon la revendication 6, comprenant en outre la transmission de l'image (1110) stockée dans la mémoire sur un réseau à un dispositif informatique distant sans nécessiter l'entrée d'un opérateur humain.

11. Procédé selon la revendication 6, comprenant en outre la transmission de l'image (1110) stockée dans la mémoire sur un réseau à un dispositif informatique distant en réponse à l'entrée d'un opérateur humain.

12. Procédé selon la revendication 6, comprenant en outre la réception d'une demande pour l'image sur un réseau au circuit de traitement à partir d'un ordinateur distant et, en réponse, la transmission de l'image sur le réseau à l'ordinateur distant.

13. Procédé selon la revendication 6, comprenant en outre l'utilisation de la caméra et du circuit de traitement pour détecter une installation incorrecte du composant jetable et la fourniture d'une alerte sonore et/ou visuelle à une interface utilisateur du dispositif médical en réponse à la détection d'une installation incorrecte du composant jetable.
